# EUROPÄISCHE PATENTSCHRIFT

(11) **EP 1 439 168 B1**
(45) Veröffentlichungstag und Bekanntmachung des Hinweises auf die Patenterteilung: **03.10.2007**
(21) Anmeldenummer: 04000328.7
(22) Anmeldetag: 09.01.2004
(51) Int. Cl.: C07C 303/32, C07C 309/42

(54) **Verfahren zur Herstellung von Acyloxybenzolsulfonaten**
Process for the preparation of acyloxybenzenesulphonates
Procédé de préparation d'acyloxybenzénésulfonates

(30) Priorität: 14.01.2003 DE 10300981
(43) Veröffentlichungstag der Anmeldung: 21.07.2004
(73) Patentinhaber: Clariant Produkte (Deutschland) GmbH, 65929 Frankfurt am Main (DE)
(72) Erfinder: Lerch, Alexander, Dr., 63571 Gelnhausen (DE); Seebach, Michael, Dr., 65719 Hofheim (DE); Mueller, Wolf-Dieter, Dr., Charlotte, NC 28277 (Mecklenburg Country (US)

(56) Entgegenhaltungen:
- EP-A- 0 164 786
- US-A- 3 503 888
- US-A- 5 069 828

## Beschreibung

Die Erfindung betrifft ein Verfahren zur Herstellung von Acyloxybenzolsulfonaten mit guten Farbqualitäten, ausgehend von Carbonsäurederivaten und Salzen einer Phenolsulfonsäure in Gegenwart eines Antioxidants.

Acyloxybenzolsulfonsäuren und ihre Salze sind seit langem bekannte Verbindungen. In Abhängigkeit von der Kettenlänge der Acylgruppe können sie als Tenside, Bleichaktivatoren oder in anderen Anwendungen Verwendung finden.
DE 666 626 beschreibt ihre tensidischen Eigenschaften und ihre allgemeine Verwendung in Waschmitteln, während Verbindungen mit 6 bis 12 Kohlenstoffatomen in der Alkylkette in Kombination mit Persalzen durch EP 98 129, EP 105 672, EP 105 673 und EP 125 641 als Bleichmittel beansprucht werden.

Zur Herstellung von Acyloxybenzolsulfonsäuren und ihren Salzen sind eine Vielzahl von Methoden beschrieben. Sie können durch Erhitzen eines Gemisches aus Trifluoressigsäureanhydrid, Natriumphenolsulfonat (SPS) und einer (C₆-C₁₉) Alkancarbonsäure erhalten werden. Nach US 4,587,054 kann diese Reaktion auch in zweistufig durchgeführt werden: Zunächst wird die Alkancarbonsäure in Gegenwart eines Überschusses von Acetanhydrid in das Anhydrid umgewandelt, anschließend wird das isolierte Anhydrid mit trockenem Phenolsulfonat umgesetzt. Diese Reaktion erfolgt bei Temperaturen von 180 bis 220°C unter Basenkatalyse. Die säurekatalysierte Umsetzung eines längerkettigen Alkansäureanhydrids mit SPS in einem aprotischen Lösemittels ist in US 4,588,532 beschrieben, die saure Katalyse (Toluolsulfonsäure und verwandte Verbindungen) erlaubt eine Reaktionsführung schon bei 120°C.
Literaturbekannt ist weiterhin die Umesterung eines (C₂-C₃)Acyloxybenzolsulfonats mit einer (C₆-C₈)Alkancarbonsäure unter gleichzeitiger Entfernung der gebildeten kurzkettigen Alkancarbonsäure. Ebenso ist die Umsetzung von Alkalimetall- oder Erdalkalimetallphenolsulfonaten mit einem C₂-C₃₁-Alkanphenylester bei 200 bis 350°C möglich.

Eine weitere Herstellungsvariante ist die Umsetzung von aliphatischen oder aromatischen Carbonsäurehalogeniden mit Salzen der Phenolsulfonsäure. Die Reaktion kann unter Schotten-Baumann-Bedingungen im wässrigen System durchgeführt werden (US 5,523,434), führt dann allerdings nur zu mäßigen Umsetzungsgraden. Vorteilhafter ist die Umsetzung von wasserfreien Salzen von Phenolsulfonsäuren in wasserfreien Medien. Als Reaktionsmedium dienen organische Lösungsmittel wie Methylenchlorid (US 35 03 888), hochsiedende Kohlenwasserstoffe (EP 220 826), Xylol oder Toluol (EP 164 786) und Trifluoressigsäure (WO 01/19 771). Gemäß US 5 069 828 wird diese Reaktion in einem aprotischen organischen Lösungsmittel in Anwesenheit eines Phasentransferkatalysators durchgeführt. Nach US Patent Anmeldung 20 020 058 824 kann diese Reaktion auch lösemittelfrei durchgeführt werden, wenn mit einem Überschuss an Säurechlorid gearbeitet wird.

Bei allen bekannten technisch verwertbaren Verfahren ergibt sich das Problem, dass oben genannte Synthesen mit einer Reihe von Nebenreaktionen eingehen, wodurch die Farbe der Endprodukte signifikant negativ beeinflusst wird.

Aufgabe der vorliegenden Erfindung ist es deshalb, ein sowohl großtechnisch als auch kontinuierlich durchführbares Verfahren zu entwickeln, das in sehr guten Ausbeuten zu möglichst einheitlichen Produkten führt, die hinsichtlich Zusammensetzung, Qualität und Farbe für den Einsatz in Wasch- und Reinigungsmitteln geeignet sind.

Überraschenderweise wurde nun gefunden, dass Acyloxybenzolsulfonate in hohen Ausbeuten und guter Farbqualität hergestellt werden können, wenn die Umsetzung eines Carbonsäurederivates mit dem Salz einer Phenolsulfonsäure in Gegenwart eines Alkylphenols oder von Tristributylphenyl-phosphit als Antioxidants erfolgt.

Gegenstand der Erfindung ist ein Verfahren zur Herstellung von Acyloxybenzolsulfonaten durch Umsetzung von Phenolsulfonaten mit Carbonsäurederivaten der Formel

R-C(O)-X,

wobei X Cl, Br oder -O-C(O)-R und R C₁-C₁₈-Alkyl, wobei diese Alkylgruppe durch eine Ester- oder Amidgruppe unterbrochen sein kann, oder C₅-C₁₁-Aryl bedeutet, dadurch gekennzeichnet, dass man die Reaktion in Gegenwart eines Di- oder Trialkylphenols oder Tris-(2,4-di-tert.-butylphenyl)phosphit oder deren Mischungen durchführt.

Bei den als Ausgangsverbindungen dienenden Phenolsulfonaten handelt es sich vorzugsweise um Verbindungen der Formel 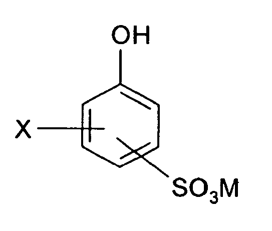 wobei X Wasserstoff, Halogen oder C₁-C₄-Alkyl und M ein Alkali- oder
Erdalkalimetall-ion bedeutet. Bevorzugt sind Natrium-ortho- oder para-Phenolsulfonate, insbesondere Natrium-para-phenolsulfonat (SPS), das herstellbedingt isomere Nebenprodukte (bis 10 %) oder andere Verunreinigungen in geringen Mengen enthalten kann.

SPS wird hergestellt durch Sulfierung von Phenol und anschließende Neutralisation. Da Na-p-Phenolsulfonat in Wasser schwer löslich ist, kann es durch Filtration, Zentrifugation oder ähnliche Prozesse aus den Reaktionsmedium isoliert werden. Das Roh-SPS wird anschließend gewaschen und weist nach der Isolierung eine hohe Reinheit und einen Wassergehalt von 15 bis 30 % auf. Für die erfindungsgemäße Reaktion mit einem Carbonsäurederivat ist es vorteilhaft das Phenolsulfonat auf eine Restfeuchte von < 0,5, vorzugsweise < 0,2 Gew.-% zu trocknen. Dieser Prozess kann kontinuierlich oder stufenweise über die Stufen des Dihydrats (Wassergehalt ca. 15 Gew.-%) und Viertelhydrats (Wassergehalt ca. 2 Gew.-%) erfolgen. Diese kann nach üblichen und an sich bekannten Verfahren geschehen, beispielsweise in einem Scheibentrockner oder Fließbetttrockner, der eine Trocknung auf eine Restfeuchte von weniger als 0,1 Gew.-% erlaubt. Während der Trocknung ist es vorteilhaft, unter ein Inertgasstrom zu arbeiten. Bei der Trocknung kann unter Vakuum oder mit gleichem Ergebnis auch ohne Vakuum gearbeitet werden.
Die Trockenzeiten können in Abhängigkeit von der verwendeten Ausrüstung zwischen 1 min und 18 h liegen, die Temperaturen zwischen 80 und 250°C. Bei dem erfindungsgemäßen Verfahren hat die thermische Vorbehandlung des getrockneten SPS keinen Einfluss auf die Ausbeute der Acylierungsreaktion und es können im Schnitt Umsätze größer 95 % erzielt werden.
In einer bevorzugten Ausführungsform wird das nach oben beschriebenem Verfahren behandelte Salz der Phenolsulfonsäure vor der Acylierung mit einer Substanz mit basischen Eigenschaften in Kontakt gebracht.
Als Substanzen mit basischen Eigenschaften kommen alle organischen oder anorganischen Verbindungen mit pkS Werten < 7 in Frage. Verwendet werden insbesondere anorganische Basen, wie Alkali- oder Erdalkalioxide, -hydroxide, -carbonate, -hydrogencarbonate, -phosphate etc. Besonders bevorzugt sind Natriumcarbonat, Natriumhydrogencarbonat und Natriumhydroxid, daneben aber auch die entsprechenden K-Salze.

Die Basen können sowohl wasserfrei, d.h. als Pulver, als Slurry, Paste oder als wässrige Lösung mit dem SPS in Kontakt gebracht werden. Dies kann direkt nach der Isolierung des SPS, d.h. nach dem Waschen des Filterkuchens oder vor bzw. während der Trocknung zum Dihydrat oder Viertelhydrat erfolgen. Alternativ kann das Inkontaktbringen auch noch während der anschließenden Trocknung zum wasserfreien SPS erfolgen. Die Zugabe kann sowohl in geeigneten Apparaturen, wie Mischern erfolgen oder auch direkt vor oder während der Trocknung selbst. In diesem speziellen Fall geschieht dies am vorteilhaftesten entweder durch Aufsprühen der gelösten Base direkt in die Trocknungsapparatur oder durch kontinuierliche Fütterung parallel zum feuchten SPS bei der Beschickung des Trockners.

Die Menge der benötigten Base liegt zwischen 0,01 und 10 Gew.-%, vorzugsweise 0,1 bis 5 Gew.-% bezogen auf das SPS in Form des Dihydrats (Wassergehalt ca. 15 Gew.-%).
Als Carbonsäure die den Carbonsäurederivaten der Formel R-C(O)-X zugrunde liegen, können lineare oder verzweigte, gesättigte oder ungesättigte Alkancarbonsäuren mit 1 bis 22 Kohlenstoffatomen verwendet werden. Beispiele hierfür sind Essigsäure, Hexansäure, Heptansäure, Octansäure, Methyloctansäure, Nonansäure, 3,3,5-Isononansäure, Decansäure, Undecansäure, Undecensäure, Laurinsäure, Myristinsäure, gehärtete Talgfettsäure, Stearinsäure, Benzoesäure oder Chlorbenzoesäure. Besonders bevorzugt werden Octansäure, Nonansäure, Isononansäure, Decansäure und Laurinsäure. Die Alkancarbonsäure kann weitere Substituenten wie Halogene, Nitro- oder Aminogruppen tragen oder durch Sauerstoffatome, Ester- bzw. Amidofunktionen unterbrochen sein. Beispiele hierfür sind n-Octylchlorameisensäure, Nonylchlorameisensäure, Octanoyloxyessigsäurechlorid, Phthalimidohexansäurechlorid und Nonanoylamidohexansäurechlorid.

Es kommen insbesondere die Carbonsäurechloride oder -bromide in Frage, wobei die Chloride bevorzugt sind. Diese können aus den entsprechenden Carbonsäuren z.B. durch Umsetzung mit Phosgen, Thionylchlorid, Phosphortrichlorid, Phosphoroxychlorid, Phosphorpentachlorid oder Phosphortribromid hergestellt werden.
Bei den Anhydriden können symmetrische oder unsymmetrische Verbindungen eingesetzt werden. Bespiele hierfür sind Acetanhydrid, Nonansäureanhydrid, Isononasäureanhydrid, Benzoesäureanhydrid, Octansäureanhydrid oder Acetylnonansäureanhydrid.

Carbonsäurederivat und Phenolsulfonat gemäß der Erfindung können vorzugsweise im Molverhältnis von 0,8 : 1 bis 2 : 1, vorzugsweise 1 : 1 bis 1,5 : 1 miteinander umgesetzt werden.

Erfindungsgemäß wird der Lösung oder Dispersion aus Phenolsulfonat vor der Acylierung mit einem Carbonsäurederivat ein oder auch mehrere Antioxidantien, wie oben definiert, zugegeben.
Besonders geeignet sind Di- und Tri-alkyl-Phenole, beispielsweise 2,4-Di-tert-butylphenol, 2,6-Di-tert-butyl-4-methyl-phenol, 2,4,6-Tri-tert-butyl-phenol, 2,6-Di-tert-butyl-4-nonyl-phenol, 6-tert-butyl-2,4-dimethyl-phenol,6-tert-butyl-2,4-dimethyl-phenol, 2,4-Dimethyl-6-nonyl-phenol, 2,4-Dimethyl-6-(1-phenyl-äthyl)-phenol, 2,4-Dimethyl-6-(1-methyl-cyclohexyl)-phenol, 2,6-Dimethyl-octadecyl-4-methyl-phenol.
Ebenso eignet sich Tris-(2,4-di-tert.-butylphenyl)-phosphit als Antioxidationsmittel.

Erfindungsgemäß zur Herstellung von Acyloxybenzolsulfonat mit guter Farbqualität besonders bevorzugte Antioxidantien sind 2,6-Di-tert-butyl-4-methyl-phenol (BHT), Tetrakis[methylen(3,5-di-tert-butylhydroxyhydrocinnamat)]methan (Hostanox^{®} 010), Octadecyl 3,5-di-tert-butyl-4-hydroxyhydrocinnamat (Hostanox 016), Tris(2,4-di-tert-butylphenyl)phosphit (Hostanox PAR24), Mischungen aus Hostanox 010 und Hostanox PAR24 (Mischungsverhältnis 1:2, Hostanox M102), Hostanox 016 und Hostanox PAR24 (Mischungsverhältnis 1:2, Hostanox M 108 oder Mischungsverhältnis 1:4, Hostanox M105), aber auch 1,3,5-Tris-(3,5-di-tert-butyl-4-hydroxy-benzyl)-[1,3,5]triazin-2,4,6-trion (Hostanox 014), Thiodiethylen bis(3,5-di-tert-butyl-4-hydroxyhydrocinnamat), N,N'-Hexamethylen bis(3,5-di-tert-butyl-4-hydroxyhydrocinnammamid), Calcium bis[monoethyl (3,5-di-tert-butyl-4-hydroxybenzyl)phosphonat], 1,3,5-Tris(3,5-di-tert-butyl-4-hydroxybenzyl)-s-triazin-2,4,6-(1 H,3H,5H)trion.
Die Antioxidantien werden in Mengen von 5 ppm bis 1000 ppm, bevorzugt 50 ppm bis 500 ppm, besonders bevorzugt 100 ppm bis 300 ppm dem Reaktionsgemisch oder den einzelnen Edukten bei Raumtemperatur oder auch bei erhöhten Temperaturen bzw. Reaktionstemperatur zugesetzt.

Die Acylierung kann in gängigen protischen oder aprotischen Lösungsmittel oder in einem Überschuss der entsprechenden Säure durchgeführt werden. Als Reaktionsmedium besonders bevorzugt werden aliphatische oder aromatische Kohlenwasserstoffe mit Siedepunkten zwischen 80 und 220°C, insbesondere 100 bis 180°C, z.B. Toluol, Xylol, Paraffine mit 8 bis 22 Kohlenstoffatomen, wie Decan, Undecan, Dodecan, Hexadecan oder Octadecan oder deren Mischungen. Besonders geeignet sind aliphatische Kohlenwasserstoffgemische wie sie als Shellsole (Shell), ISOPAR G und ISOPAR 4 (ESSO) kommerziell verfügbar sind. Die Löslichkeit des SPS in diesem Reaktionsmedium liegt häufig unter 1 %.

Ein zusätzlicher Katalysator ist normalerweise nicht notwendig, kann jedoch in einzelnen Fällen Vorteile bieten. Bevorzugt sind offenkettige oder cyclische tertiäre Amine oder Carbonsäureamide (wie in DE 101 39 663.5 beschrieben), Phasentransferkatalysatoren oder saure Katalysatoren wie p-Toluolsulfonsäure. Das Molverhältnis des eingesetzten Katalysators zum Phenolsulfonat beträgt 0,0001: 1 bis 0,02 : 1, vorzugsweise 0,005 : 1 bis 0,012 : 1.

Die Acylierungsreaktion wird bei Temperaturen zwischen 60 und 200°C, insbesondere zwischen 100 und 150°C durchgeführt. Das während der Reaktion entstehende Gas wird abgeleitet, ggf. wird die Reaktion mit einem inerten Gasstrom aus Stickstoff oder Argon überlagert. Die Reaktion wird im Sinne einer heterogenen Reaktion (Slurry) durchgeführt, da weder das Phenolsulfonat noch das entstehende Acyloxybenzolsulfonat eine nennenswerte Löslichkeit im Reaktionsmedium aufweisen. Die Reaktionszeit richtet sich nach den Reaktionsbedingungen und kann zwischen 10 min und 5 h, vorzugsweise 30 bis 120 min betragen.

In einer besonderen Ausführungsform kann die erfindungsgemäße Reaktion kontinuierlich durchgeführt werden. Hierzu besonders geeignet sind Kesselkaskaden bzw. Rohrreaktoren, wie sie dem Fachmann bekannt sind.
Nach beendeter Reaktion wird das Reaktionsprodukt mittels konventioneller Trennmethoden isoliert. Hierzu eignen sich Zentrifugen, Filterapparate. Zur völligen Abtrennung des Katalysators empfiehlt es sich, das feste Reaktionsprodukt ein oder mehrmals mit dem Reaktionsmedium auszuwaschen. Die Mutterlauge kann ohne weitere Reinigung für die nächsten Reaktionen verwendet bzw. cycliert werden. Das gebildete Acyloxybenzolsulfonat fällt in hohen Ausbeuten in Form eines weißen Pulvers an, das durch konventionelle Trocknung isoliert werden kann.

Die nach dem oben beschriebenen Verfahren in Gegenwart der zuvor definierten Antioxidantien hergestellten Acyloxybenzolsulfonate zeichnen sich durch eine signifikant bessere Farbqualität im Vergleich zu Produkten aus, die in Abwesenheit von Antioxidationsmittel synthetisiert wurden.

### Beispiele

### Synthesemethode I (ohne BHT):

98,1 g (0,5 mol) p-Phenolsulfonat, Na-Salz (16 h bei 130°C/50 mbar getrocknet, behandelt mit NaHCO3) wurden in 146.77 g Isopar G (aliphatischer Kohlenwasserstoff) vorgelegt und auf 140°C geheizt. Innerhalb von 30 min. wurden bei 140°C 114.9 g (0.65 mol) Nonansäurechlorid zugetropft, 2 h bei 140°C nachgerührt und auf 80°C abgekühlt. Der Feststoff wurde bei 600 mmHg über einen Büchner-Trichter (Pannevis) abgenutscht, mit 84 g Isopar G gewaschen und das Lösungsmittel im Vakuum entfernt. Die Trocknung des Filterkuchens erfolgte im Rotationsverdampfer bei 200°C und 50 mbar über einen Zeitraum von 10 Min. bzw. 20 Min.

### Synthesemethode II (mit Antioxidant):

98,1 g (0,5 mol) p-Phenolsulfonat, Na-Salz (16 h bei 130°C/50 mbar getrocknet, behandelt mit NaHCO3) in 146,77 g Isopar G (aliphatischer Kohlenwasserstoff) und 3,43 g (entspricht 250 ppm BHT) einer Lösung aus 1,2607 g 2,6-Di-tert.-butyl-4-methylphenol, (BHT) in 46,2418 g Isopar G) wurden vorgelegt und auf 140°C geheizt. Innerhalb von 30 min. wurden bei 140°C 114,9 g (0.65 mol) Nonansäurechlorid zugetropft, 2 h bei 140°C nachgerührt und auf 80°C abgekühlt. Der Feststoff wurde bei 600 mmHg über einen Büchner-Trichter (Pannevis) abgenutscht, mit 84 g Isopar G gewaschen und das Lösungsmittel im Vakuum entfernt. Die Trocknung des Filterkuchens erfolgte im Rotationsverdampfer bei 200°C und 50 mbar über einen Zeitraum von 10 Min. bzw. 20 Min.

Die Herstellung von Nonanoyloxybenzolsulfonat mit dem Antioxidant Tris(2,4-di-tert-butylphenyl)phosphit (Hostanox PAR24), erfolgte in analoger Weise mit Einsatzmengen an Antioxidant von 200 ppm und 400 ppm.

Die Farbqualität wurde anhand der Hazenfarbzahl mittels eines Farbmessgerätes LICO 300 nach DIN 53995 bestimmt. Es wurden folgende Werte gemessen:

| Synthesemethode | Trockenzeit [min] | Hazen-Farbzahl [mg Pt/min] |
|---|---|---|
| I (ohne BHT) | 10 | 63 |
| I (ohne BHT) | 20 | 61 |
| II (mit BHT) | 10 | 37 |
| II (mit BHT) | 20 | 40 |

Diese Werte für die Farbzahl zeigen, dass man durch den Zusatz von Antioxidantien gemäß der vorliegenden Erfindung Produkte erhält, die wesentlich weniger farbige Verunreinigungen enthalten im Vergleich zu solchen Produkten, bei deren Herstellung keine Antioxidantien eingesetzt werden.

Das auf diese Weise gewonnene Acyloxybenzolsulfonat kann als Tensid oder Persalzaktivator in Wasch- und Reinigungsmittel wie pulverförmigen Vollwaschmitteln, Fleckensalzen oder pulverförmigen Maschinengeschirrspülmitteln eingesetzt werden. Zur Erhöhung der Lagerstabilität in diesen Formulierungen kann es, wie dem Fachmann bekannt, in eine granulare Form überführt werden.

## Patentansprüche

1. Verfahren zur Herstellung von Acyloxybenzolsulfonaten durch Umsetzung von Phenolsulfonaten mit Carbonsäurederivaten der Formel
R-C(O)-X,
wobei X Cl, Br oder-O-C(O)-R und R C₁-C₁₈-Alkyl, wobei diese Alkylgruppe durch eine Ester- oder Amidgruppe unterbrochen sein kann, oder C₅-C₁₁-Aryl bedeutet, **dadurch gekennzeichnet, dass** man die Reaktion in Gegenwart eines Di- oder Trialkylphenols oder Tris-(2,4-di-tert.-butylphenyl)phosphit oder deren Mischungen durchführt.

2. Verfahren nach Anspruch 1, **dadurch gekennzeichnet, dass** man als Antioxidant 2,6-Di-tert.-butyl-4-methyl-phenol einsetzt.

3. Verfahren nach Anspruch 1, **dadurch gekennzeichnet, dass** man die Antioxidantien in Mengen von 5 ppm bis 1000 ppm, bevorzugt 50 ppm bis 500 ppm, besonders bevorzugt 100 ppm bis 300 ppm einsetzt.

4. Verfahren nach Anspruch 1, **dadurch gekennzeichnet, dass** man die Umsetzung mit einem Salz einer Phenolsulfonsäure durchführt, das einen Wassergehalt von weniger als 0,5 Gew.-% aufweist.

5. Verfahren nach Anspruch 1, **dadurch gekennzeichnet, dass** man die Umsetzung mit einem Salz einer Phenolsulfonsäure durchführt, das einen Wassergehalt von weniger als 0,2 Gew.-% aufweist.

6. Verfahren nach Anspruch 1, **dadurch gekennzeichnet, dass** man das Salz der Phenolsulfonsäure vor der Umsetzung mit dem Carbonsäurederivat mit einer Substanz mit basischen Eigenschaften in Kontakt bringt, die einen pKS-Wert von unter 7 hat.

## Claims

1. A process for preparing acyloxybenzenesulfonates by reacting phenolsulfonates with carboxylic acid derivatives of the formula
R-C(O)-X,
where X is Cl, Br or -O-C(O)-R and R is C₁-C₁₈ alkyl, where this alkyl group may be interrupted by an ester group or amide group, or C₅-C₁₁ aryl, which comprises carrying out the reaction in the presence of a di- or tri-alkylphenol or tris(2,4-di-tert-butylphenyl) phosphite or mixtures thereof.

2. The process as claimed in claim 1, wherein 2,6-di-tert-butyl-4-methylphenol is used as antioxidant.

3. The process as claimed in claim 1, wherein the antioxidant is used in amounts of from 5 ppm to 1 000 ppm, preferably from 50 ppm to 500 ppm, more preferably from 100 ppm to 300 ppm.

4. The process as claimed in claim 1, wherein the reaction is conducted with a salt of a phenolsulfonic acid which has a water content of less than 0.5% by weight.

5. The process as claimed in claim 1, wherein the reaction is conducted with a salt of a phenolsulfonic acid which has a water content of less than 0.2% by weight.

6. The process as claimed in claim 1, wherein the salt of the phenolsulfonic acid is contacted, prior to the reaction with the carboxylic acid derivative, with a substance having basic properties which has a pKa of less than 7.

## Revendications

1. Procédé pour la préparation d'acyloxybenzènesulfonates par réaction de phénolsufonates avec des dérivés d'acides carboxyliques de formule
R-C(O)-X,
où X représente un atome de Cl, de Br ou un groupe O-C(O)-R et R représente un groupe alkyle en C₁-C₁₈, ce groupe alkyle pouvant être interrompu par un groupe ester ou amide, ou représente un groupe aryle en C₅-C₁₁, **caractérisé en ce que** la réaction est mise en oeuvre en présence d'un di- ou trialkylphénol ou d'un phosphite de tris-(2,4-di-tert-butylphényle) ou de leurs mélanges.

2. Procédé selon la revendication 1, **caractérisé en ce qu'**on utilise en tant qu'antioxydant le 2,6-di-tert-butyl-4-méthyl-phénol.

3. Procédé selon la revendication 1, **caractérisé en ce qu'**on utilise les antioxydants en quantités de 5 ppm à 1000 ppm, de préférence de 50 ppm à 500 ppm, de manière particulièrement préférée de 100 ppm à 300 ppm.

4. Procédé selon la revendication 1, **caractérisé en ce que** la réaction est mise en oeuvre avec un sel d'un acide phénolsulfonique qui présente une teneur en eau inférieure à 0,5 % en masse.

5. Procédé selon la revendication 1, **caractérisé en ce que** la réaction est mise en oeuvre avec un sel d'un acide phénolsulfonique qui présente une teneur en eau inférieure à 0,2 % en masse.

6. Procédé selon la revendication 1, **caractérisé en ce que** le sel de l'acide phénolsulfonique est mis en contact, avant la réaction avec le dérivé de l'acide carboxylique, avec une substance ayant des propriétés basiques, qui a une valeur de pKS inférieure à 1.
